# EUROPEAN PATENT SPECIFICATION

(11) **EP 0 801 131 B1**
(45) Date of publication and mention of the grant of the patent: **21.02.2007**
(21) Application number: 95941892.2
(22) Date of filing: 26.12.1995
(51) Int. Cl.: C12N 9/86, C12N 15/00, C12P 41/00

(54) **PROCESS FOR PRODUCING D-N-CARBAMOYL-ALPHA-AMINO ACID**
VERFAHREN ZUR HERSTELLUNG EINER D-N-CARBAMOYL-ALPHA-AMINOSÄURE
PROCEDE DE FABRICATION DE L'ACIDE D-N-CARBAMYLE-ALPHA-AMINE

(30) Priority: 28.12.1994 JP 32686594
(43) Date of publication of application: 15.10.1997
(60) Divisional application: 05021429.5
(73) Proprietor: KANEGAFUCHI KAGAKU KOGYO KABUSHIKI KAISHA, Kita-ku Osaka-shi Osaka-fu 530 (JP)
(72) Inventor: NANBA, Hirokazu, Kakogawa-shi, Hyogo-ken (JP); YAJIMA, Kazuyoshi, Akashi-shi, Hyogo-ken (JP); TAKANO, Masayuki, Kakogawa-shi, Hyogo-ken (JP); YAMADA, Yukio, Kakogawa-shi, Hyogo-ken (JP); IKENAKA, Yasuhiro, Akashi-shi, Hyogo-ken (JP); TAKAHASHI, Satomi, Kobe-shi, Hyogo-ken (JP)
(74) Representative: Polz, Leo
(86) International application number: PCT/JP1995/002688
(87) International publication number: WO 1996/020275

(56) References cited:
- EP-A- 0 261 836
- EP-A- 0 643 133
- EP-A- 0 677 585
- WO-A-92/10579
- WO-A-92/22643
- WO-A-94/00577
- WO-A-96/00296
- JP-A- 4 325 093
- JP-A- 62 087 089
- PATENT ABSTRACTS OF JAPAN vol. 012, no. 230 (C-508), 29 June 1988 (1988-06-29) & JP 63 024894 A (AJINOMOTO CO INC), 2 February 1988 (1988-02-02)
- PATENT ABSTRACTS OF JAPAN vol. 015, no. 341 (C-0863), 29 August 1991 (1991-08-29) & JP 03 133386 A (KANEGAFUCHI CHEM IND CO LTD), 6 June 1991 (1991-06-06)
- BIOSCIENCE BIOTECHNOLOGY AND BIOCHEMISTRY, Vol. 58, No. 9, (9-1994), MUKOHARA Y., "A Thermostable Hydantoinase of Bacillus Stearothermophilus NS1122A", pages 1621-1626.

## Description

### Field of the Invention

The present invention relates to a process for producing D-N-carbamoyl-α-amino acids. More specifically, it relates to a process for producing D-N-carbamoyl-α-amino acids utilizing a novel transformant having a gene for an enzyme which converts 5-substituted hydantoins into corresponding D-N-carbamoyl-α-amino acids by asymmetric cleavage hydrolysis (hereinafter referred to as hydantoinase).

### Backgrounds of the Invention

Optically-active D-N-carbamoyl-α-amino acids can be converted into corresponding D-α-amino acids and optically-active D-α-amino acids are important compounds as intermediate compounds for drugs. In particular, examples of industrially useful compounds include D-phenylglycine and D-(p-hydroxyphenyl)glycine as intermediate compounds for the production of semisynthetic penicillins and semisynthetic cephalosporins.

For the production of D-N-carbamoyl-α-amino acids, processes utilizing microbial enzymatic reactions have been known to convert 5-substituted hydantoins into corresponding D-N-carbamoyl-α-amino acids by asymmetric cleavage hydrolysis and such processes are described in JP-A 53-91189, JP-A 53-44690, JP-A 53-69884, JP-A 53-133688 and the like.

In addition, a gene relating to hydantoinase which is an enzyme for conventing a 5-substituted hydantoin into the corresponding D-N-carbamoyl-α-amino acid is obtained
from thermophilic microorganisms and it is inserted into mesophilic microorganisms to produce transformant microorganisms having hydantoinase activity. Furthermore, in WO94/00577, a hydantoinase gene fragment is isolated from a microorganism belonging to the genus Agrobacterium and introduced into Escherichia coli or a microorganism of the genus Agrobacterium to express the activity.

### Objects of the Invention

In the above-described processes utilizing microbial enzymatic reactions, there are such drawbacks that yields of the enzyme by microorganisms known to be its source is insufficient and, further, culture mediums are expensive.

In addition, with regard to the transformant microorganisms disclosed in JP-A 62-87069, the improvement of the enzymatic activity is only several times in comparison with sporulating thermophilic microorganisms having hydantoinase activity and yields of the enzyme is yet insufficient.

Furthermore, there is no example in the prior art that a 5-substituted hydantoin is subjected to asymmetrical cleavage hydrolysis by using the above-described transformant microorganism to produce and accumulate the corresponding D-N-carbamoyl-α-amino acid.

The present invention is to solve these problems and is directed to the creation of a microorganism having very high enzyme productivity and to the production of D-N-carbamoyl-α-amino acids efficiently by using the enzyme source thus obtained.

### Summary of the Invention

JP-A 62-87089 and WO94/00577 disclose the method for obtaining hydantoinase producer microorganisms by using recombinant DNA techniques. However, the source microorganisms for supplying hydantoinase genes are completely different from microorganisms used for obtaining hydantoinase genes in the present invention. In addition, the nucleotide sequences and amino acid sequences of the hydantoinase genes are considerably different from those of the present invention.

The present invention is to provide a process for producing a D-N-carbamoyl-α-amino acid which comprises reacting a 5-substituted hydantoin in an aqueous medium with hydantoinase produced by a transformant obtained by transforming a host microorganism such as a microorganism belonging to the genus Escherichia, Pseudomonas, Flavobacterium, Bacillus, Serratia, Agrobacterium, Corynebacterium or Brevibacterium with a recombinant DNA of a DNA fragment containing a gene for hydantoinase derived from Bacillus sp. KNK245 (FERM BP-4863), and a vector DNA.

A transformant having very high hydantoinase productivity such as that obtained in the present invention has not been known heretofore in the prior art and the extremely efficient and economical production of D-N-carbamoyl-α-amino acids become possible by utilizing the reaction of the enzyme of the transformant.

Hereinafter, the present invention will be described in detail with reference to the accompanying drawings.

### Brief Explanation of the Drawings

Fig. 1 is a restriction enzyme map of plasmid pAH1043.
Fig. 2 is a restriction enzyme map of plasmid pTH102.
Fig. 3 is a restriction enzyme map of plasmid pTH103.
Fig. 4 is a restriction enzyme map of plasmid pTH104.
Fig. 5 is a restriction enzyme map of plasmid pPHD301.
Fig. 6 is a restriction enzyme map of plasmid pTHB301.

### Detailed Explanation of the Invention

A DNA fragment containing the gene to be used in the present invention can be obtained from Bacillus sp. KNK245, Agrobacterium sp. KNK712 and Pseudomonas sp. KNK003A have been deposited at National Institute of Bioscience and Human-Technology, Agency of Industrial Science & Technology (NIBH), Ministry of International Trade & Industry by the present applicant under accession numbers of FERM BP-1900 and FERM BP-3181, respectively and are known strains which described in detail in WO92/10579. Bacillus sp. KNK245 is a strain newly found by the present inventors and has been deposited at NIBH as of November 2, 1994 under the accession number of FERM BP-4863.

Microbiological characteristics of Bacillus sp. KNK245 are as follows.

| | | |
|---|---|---|
| Bacillus sp. KNK245 | | |
| Form | | rods, filamentous |
| Size | | 0.5-0.8 x 3-10 (µm) |
| Gram stain | | + |
| Spores | | + |
| Motility | | - |
| Growth temperature | | 37-60°C |
| Growth pH | | 5.0-8.9 |
| Growth in 5% NaCl | | - |
| Growth in 7% NaCl | | - |
| Growth in 0.02% azide | | - |
| Growth in 0.001% lysozyme | | - |
| Nitrate reduction | | - |
| Starch hydrolysis | | + |
| Casein decomposition | | + |
| Tyrosine decomposition | | - |
| Catalase | | - |
| Oxidase | | + |
| Indole production | | - |
| Production of acids from carbohydrates | | |
| | Mannose | (+) |
| | Xylose | - |
| | Arabinose | + |
| | Rhamnose | - |
| | Glucose | + |
| | Fructose | + |
| | Raffinose | - |
| | Sucrose | + |
| | Maltose | + |
| | Lactose | - |

For obtaining a hydantoinase gene from these strains, normally, the following procedure is carried out.

Firstly, genomic DNA is extracted from a microbial cell and then the DNA is partially hydrolyzed with a suitable restriction enzyme, for example, Sau3AI or the like. The resulting fragments are ligated to vector DNA to obtain recombinant DNA molecules having various genomic DNA fragments as a gene library (see JP-A 58-126789 and U.S. Patent No. 4,237,224).

Then, a recombinant containing the desired gene is selected from this gene library. For example, the selection can be carried out by detection of an expressed protein using its enzymatic activity as an indication, or the selection can be carried out by analyzing the N-terminal sequence of a protein, synthesizing a corresponding DNA probe and detecting the presence of the desired gene by colony hybridization or the like. In addition, the desired gene can be obtained by colony hybridization or polymerase chain reaction (PCR) method using DNA primers which are synthesized suitable portions of a known hydantoinase base sequence. Once the desired gene is obtained, its nucleotide sequence is analyzed. In addition, a hydantoinase producer microorganism and a recombinant containing this enzyme gene are cultivated. The resultant enzyme is purified and the molecular weight of the resultant protein is determined. At the same time, the amino acid sequence about its N-terminal region is determined with a gas phase protein sequencer or the like.

Then, by comparison of these DNA nucleotide sequences and N-terminal amino acid sequences, the initiation site for translation of the nucleotide sequence portion encoding a hydantoinase protein into the protein is determined and, by considering the relation to the molecular weight of the protein, it is confirmed that the enzyme protein is encoded in the gene portion extending from this site to the termination codon, thereby verifying the desired gene (Molecular Cloning, A Laboratory Manual, 2nd Ed., Cold Spring Harbor Laboratory Press, Chapters 4 and 13). Thus, DNA fragments of SEQ ID Nos. 1 and 2 were obtained from Agrobacterium sp. KNK712 and Pseudomonas sp. KNK003A. As is well known, the gene encoding the enzyme thus obtained and/or DNA fragment containing it are equivalent to DNA fragments having another nucleotide sequence which encodes an amino acid sequence corresponding to the above gene and/or DNA fragment because one amino acid usually corresponds to a plurality of base codons.

As the vector to be used in the present invention, plasmids, phages or derivatives thereof can be used, which are derived from microorganisms and can be autonomously grown in cells of bacteria belonging to the genus Escherichia, Pseudomonas, Flavobacterium, Bacillus, Serratia, Corynebacterium or Brevibacterium.

For example, host-vector systems described in "Guidelines on Recombinant DNA Experiments, -Commentation, Q and A-" (the Life Science Section of the Research Development Office in the Science and Technology Agency, Ed., revised September 16, 1987), pages 25 to 27 and 36 to 38 can be used. In addition, recombinant DNA having a translation initiation site linked to a suitable position of a vector which has been modified to have a strong structural promoter can be used for the purpose of increasing the amount of enzyme to be produced. Both ends of these fragments are cleaved with a restriction enzyme to produce a recombinant with an appropriate vector. A hydantoinase producer recombinant microorganism can be produced by directly transforming a microorganism belonging to the genus Escherichia, Pseudomonas, Flavobacterium, Bacillus, Serratia, Agrobacterium, Corynebacterium or Brevibacterium.

A hydantoinase producer recombinant microorganism can also be obtained without direct transformation of a microorganism of the above-described genus; that is, the desired gene is once cloned in a host vector system using other microorganisms such as Escherichia coli, after which recombinant DNA with an appropriate vector is produced.

The description of the following documents can be widely applied to the production of recombinants: the specification of U.S. Patent No. 4,237,244 to S. N. Cohen et al.; "Idenshi-sousa Jikken-hou (Experimental Method of Gene Manipulation)" [Ed., Yasuyuki TAKAGI, Kohdan-sha Scientific (1980)]; Method in Enzymology, 68, Recombinant DNA [Ed., Ray Mv, Academic Press (1979)], the specification of JP-A 58-126789 and the like.

The desired gene is cloned and unnecessary DNA thereof is removed. Recombinant DNA having its translation initiation site linked to a suitable position of a vector which has been modified to have a strong structural promoter can be used for transformation of the above host cell to increase the amount of the enzyme to be produced.

The transformant is cultivated in a conventional nutrient culture medium to express the introduced recombinant DNA. When a character derived from gene DNA or vector DNA is conferred to the recombinant DNA, an appropriate component can be added to the culture medium according to a particular character.

To take the transformant thus obtained as a source of enzyme supply, it can be cultivated by using the conventional culture medium. If necessary, it is also possible to carry out a treatment for enzyme induction such as an addition of hydantoin compounds, uracil, isopropyl-l-thio-β-D-galactosides (IPTG) or the like and a temperature increase. Usually, the culture medium used for cultivating the transformant can be a conventional culture medium containing carbon sources, nitrogen source and inorganic ions. In many cases, desired results can be obtained by further adding organic trace nutrients such as vitamins, amino acids and the like. As the carbon sources, carbohydrates such as glucose and sucrose, organic acids such as acetic acid, alcohols and the like can be conveniently used. As the nitrogen sources, ammonia gas, ammonia water, ammonium salts and the like can be used. As the inorganic ions, phosphate ion, magnesium ion, potassium ion, iron ion, manganese ion, cobalt ion, nickel ion, copper ion, aluminum ion, molybdenum ion and the like can be used.

If cultivation is carried out under aerobic conditions for 1 to 10 days, while adjusting the pH and temperature to an appropriate range of 4 to 8 and 25 to 60°C, respectively, desired results can be obtained.

The enzymatic activity of an enzyme produced by the transformant can be exhibited in the form of, for example, a culture solution of the transformant, its microbial cells, the enzyme extracted from the microbial cells, immobilized microbial cells and the like.

As the microbial cells, any of the culture solution as it is after completion of cultivation, microbial cells separated from the culture solution, washed microbial cells and the like can be used. As the treated microbial cells, lyophilized microbial cells, acetone-dried microbial cells, microbial cells brought into contact with toluene or a detergent, lysozyme treated microbial cells, ultrasonicated microbial cells, mechanically ground microbial cells and the like can be used. In addition, enzyme extracts having hydantoinase activity obtained from these treated microbial cells, these immobilized microbial cells, insolubilized treated microbial cells, enzyme proteins fixed on a support for immobilization (e.g., anion exchange resin) and the like can be used. For an immobilization method, for example, the specification of JP-A 63-185382 and the like can be referred to.

As the support used for immobilization, suited are phenol-formaldehyde anion exchange resins such as Duolite A568 or DS17186 (Rohm & Haas Co.: registered trade mark); and various anion exchange resins containing various amines, ammonium salts, or functional groups of the diethanolamine type, for example, polystylene resins such as Amberlite IRA935, IRA945, IRA901 (Rohm & Haas Co.: registered trade mark), Lewatit OC1037 (Bayer A.G.: registered trade mark) and Diaion EX-05 (Mitsubishi Chemical Industries, Ltd.: registered trade mark). Other supports such as DEAE-cellulose can also be used.

Further, in order to obtain stronger and more stable adsorption of enzymes, usually, cross-linking agents are used, the preferred examples thereof being glutaraldehyde. As the enzyme to be used, not only purified enzymes, but also those with different degrees of purification such as partially purified enzymes, suspensions of disintegrated microbial cells and cell-free extracts can be used. The preparation of immobilized enzymes can be carried out according to a conventional method, for example, by adsorbing enzymes on a support, followed by cross-linking treatment.

The 5-substituted hydantoin to be used as a substrate of the enzymatic reaction of the present invention is not specifically limited and can be any compound normally used in this kind of reaction. Examples thereof include that represented by the general formula (1):

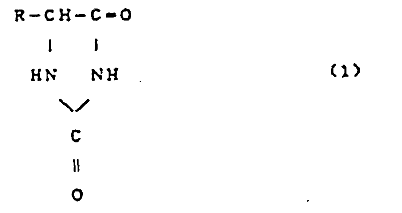

The substituent R in the compound of the formula (1) can be selected in a wide range. In order to provide industrially useful products such as intermediate compounds of drugs, preferably, R is phenyl, phenyl substituted with hydroxy, alkyl, substituted alkyl, aralkyl or thienyl. In the case of phenyl substituted with hydroxy, the number of hydroxy may be one or more and they can be located at any of o-, m- and p-positions, with the typical example thereof being p-hydroxyphenyl. The alkyl group is such a group of 1 to 4 carbon atoms that the corresponding D-N-carbamoyl-α-amino acid is D-N-carbamoyl-alanine, D-N-carbamoyl-valine, D-N-carbamoyl-leucine, D-N-carbamoyl-isoleucine or the like. The substituted alkyl group is such an alkyl group of 1 to 4 carbon atoms substituted with hydroxy, alkylthio, carboxyl, amino, phenyl, phenyl substituted with hydroxy, amido or the like that the corresponding D-N-carbamoyl-amino acid is D-N-carbamoyl-serine, D-N-carbamoyl-threonine, D-N-carbamoyl-methionine, D-N-carbamoyl-cysteine, D-N-carbamoyl-asparagine, D-N-carbamoyl-glutamine, D-N-carbamoyl-tyrosine, D-N-carbamoyl-tryptophane, D-N-carbamoyl-aspartic acid, D-N-carbamoyl-glutamic acid, D-N-carbamoyl-histidine, D-N-carbamoyl-lysine, D-N-carbamoyl-arginine, D-N-carbamoyl-citrulline or the like. The aralkyl group is such a group having 7 to 8 carbon atoms, for example, benzyl or phenethyl, that the corresponding D-N-carbamoyl-α-amino acid is D-N-carbamoyl-phenylalanine or the like.

As the aqueous medium, those containing water, buffers or organic solvents such as ethanol can be used. Further, when necessary, nutrients required for the growth of microorganisms, antioxidants, detergents, coenzymes, hydroxylamines, metals and the like can also be added to the aqueous medium.

In the case where, while cultivating the microbial cells of the above microorganisms in a water-soluble medium, the microbial cells are brought into contact with a particular 5-substituted hydantoin, an aqueous medium containing not only the 5-substituted hydantoin but also nutrients required for the growth of microorganisms such as carbon sources, nitrogen sources and inorganic ions is used. If organic trace nutrients such as vitamins and amino acids are further added thereto, satisfactory results can be obtained in many cases. As the carbon sources, carbohydrates such as glucose, sucrose; organic acids such as acetic acid; alcohols and the like are conveniently used. As the nitrogen sources, ammonia gas, ammonia water, ammonium salts and the like can be used. As the inorganic ions, phosphate ion, magnesium ion, potassium ion, iron ion, manganese ion, cobalt ion, nickel ion, copper ion, aluminum ion, molybdenum ion and the like can be used.

The cultivation is carried out under aerobic conditions, while adjusting the pH and temperature to an appropriate range of 4 to 8 and 25 to 60°C, respectively. If the cultivation is carried out for 1 to 10 days, 5-substituted hydantoins are converted into only D-N-carbamoyl-α-amino acids with high efficiency.

On the other hand, in the case where the culture solution of the above-microorganisms is allowed as it is to react with cultivated microbial cells, treated microbial cells, enzyme extracts, immobilized microbial cells, insolubilized microbial cells or fixed enzyme proteins in an aqueous medium containing a 5-substituted hydantoin dissolved or suspended, the reaction mixture can be allowed to stand for some time or stirred, while maintaining at an appropriate temperature of 10 to 80°C and pH of 4 to 9.5. Thus, if 5 to 100 hours have run their course, D-specific hydrolysis reaction of the substrate by hydantoinase and chemical racemization of the substrate proceed and any of D-, DL- or L-5-substituted hydantoins are converted into the corresponding D-N-carbamoyl-α-amino acids to accumulate a large amount of the amino acids in the aqueous medium. In addition, 5-substituted hydantoins can be added in separate portions with the progress of reaction.

The produced D-N-carbamoyl-α-amino acid can be isolated and purified by a conventional separation method.

D-α-amino acids can be easily obtained by using the isolated and purified D-N-carbamoyl-α-amino acids obtained by the above-described method or the reaction mixtures obtained by the hydantoinase reaction as they are by the action and converting the D-N-carbamoyl-α-amino acids into the corresponding D-α-amino acids chemically or enzymatically with enzymes having decarbamylase activity.

The following Examples further illustrate the present invention in detail.

### Example 1

### Isolation of microorganism having hydantoinase activity from soil

After suspending 0.5 g of a sample of soil in 2 ml of a physiological saline, the suspension was allowed to stand and one drop of the supernatant was smeared on an isolation agar plate medium (1.0% of meat extract, 1.0% of peptone, 1.0% of yeast extract, 0.3% of sodium chloride, 2.0% of agar; pH 7.5). This was cultivated at 50°C for 2 days and the colony obtained was transferred to the same isolation plate medium to which 0.1% of uracil and 20 ppm of manganese chloride and then further cultivated at 50°C for 1 day. The microbial cells thus obtained were suspended in 100 µl of a reaction substrate solution [DL-(p-hydroxyphenyl)hydantoin 30 mM, sodium sulfite 0.1%, carbonate buffer 50 mM] and they were reacted at 50°C for 2 hours. Then, the reaction mixture was spotted on a TLC plate and developed with a developing solvent of butanol : acetic acid : water (4 : 1 : 1), followed by developing color with a 10% solution of p-dimethylaminobenzaldehyde in conc. hydrochloric acid and detecting a yellow spot to select microbial strains producing N-carbamoyl-p-hydroxyphenylglycine. Thus, among 2740 colonies, Bacillus sp. KNK245 (FERM BP-4863) having high hydantoinase activity was isolated.

### Example 2

### N-Terminal amino acid sequencing of hydantoinase derived from Bacillus sp. KNK245

A loopful of Bacillus sp. KNK245 was inoculated into 100 ml of a seed culture medium (1.0% of meat extract, 1.0% of peptone, 0.5% of yeast extract; pH 7.5) in a 500 ml Sakaguchi flask and cultivated at 55°C for 19 hours. Then, 2% thereof was inoculated into 500 ml of a main culture medium (1.0% of meat extract, 1.0% of peptone, 0.5% of uracil, 20 ppm of manganese chloride tetrahydrate; pH 7.5) in a 2 liter Sakaguchi flask and cultivated at 55°C for 19 hours. Microbial cells were collected from 9 liters of the culture broth thus obtained by centrifugation. They were suspended in 0.2 M Tris-HCl (pH 8.0) with 20 mM manganese sulfate, ultrasonicated and centrifuged to obtain a cell-free extract. Then, the extract was purified up to 32 times by means of ammonium sulfate precipitation fractionation, DEAE-Sepharose and phenyl-Sepharose. Further, hydantoinase crystals were obtained by using ammonium sulfate. The crystals were dissolved in water and analyzed by using 470 A Model Gas Phase Protein Sequencer (manufactured by Applied Biosystems). As a result, it was found that the hydantoinase had the amino acid sequence of: 
1                   5                   10

Met-Lys-Lys-Ile-Ile-Lys-Asn-Gly-Thr-Ile-Val-Thr-
 at its N-terminal.

### Reference Example 1

### To obtain plasmid containing gene for hydantoinase derived from Agrobacterium sp. KNK712 (FERM Hp-1900)

It was found that a hydantoinase gene was located on the DNA fragment cloned in the plasmid pAHD101 described in WO92/10579 by a study according to the following method. Escherichia coli JM109 was transformed with pAHD101 according to a conventional method, inoculated into 50 ml of L-broth (10 g/liter of peptone, 5 g/liter of yeast extract, 5 g/liter of sodium chloride; pH 7.0) containing 100 mg/liter of ampicillin and cultivated at 37°C for 16 hours. Then, 50 ml of the culture broth was collected, followed by removing the supernatant, suspending the microbial cells in 50 ml of a substrate solution (0.5% of 5-(p-hydroxyphenyl)hydantoin, 0.05% of Triton X-100, 0.05 M phosphate buffer; pH 8.7) and reacting at 37°C for 3 hours in a stream of nitrogen. The reaction mixture was spotted on a TLC plate, developed and subjected to color development with a solution of p-dimethylaminobenzaldehyde in hydrochloride to detect a spot of D-N-carbamoyl-(p-hydroxyphenyl)glycine in agreement with an authentic sample. In view of the above, it was confirmed that the transformant of pAHD101 expresses a gene encoding a hydantoinase.

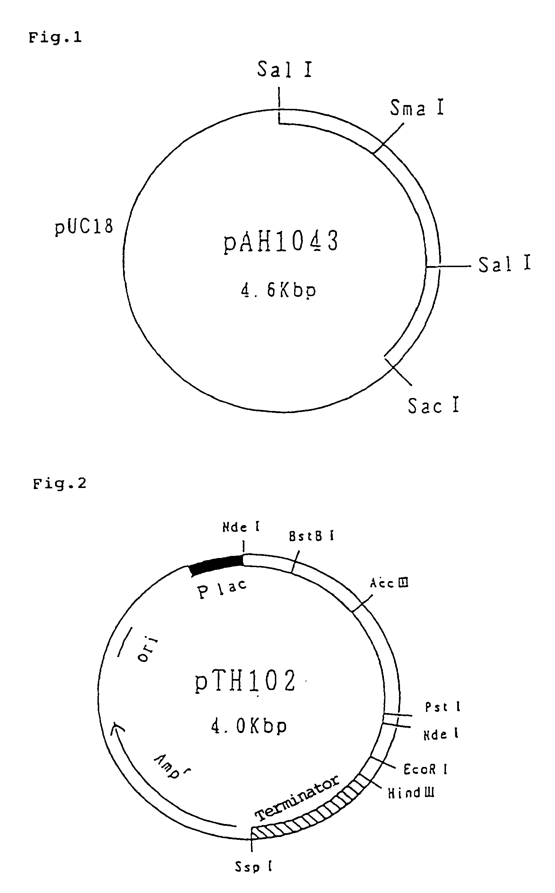

A 2.1 kbp fragment obtained by cleaving the plasmid pAHD101 with SacI and SalI to shorten the inserted fragment was ligated to pUC18 fragment cleaved by SacI and SalI to obtain the plasmid pAH1043 (see Fig. 1).

In addition, the nucleotide sequencing of the hydantoinase gene was carried out by using DNA sequence kit (manufactured by Applied Biosystems). The results are shown in SEQ ID No. 1

Escherichia coli HB101 was transformed with the plasmid pAH1043 to obtain a transformant, Escherichia coli HB101 pAH1043 (FERM BP-4865).

### Example 3

### Preparation of recombinant DNA of genomic DNA of Bacillus sp. KNK245 and vector DNA

Bacillus sp. KNK245 (FERM BP-4863) was cultivated in 2 liters of a broth (10 g/liter of meat extract, 10 g/liter of peptone, 5 g/liter of yeast extract; pH 7.5) at 45°C for 24 hours and microbial cells were collected. Genomic DNA was extracted from the microbial cells obtained according to Marmur method. Ten units of BamHI was added to 1 µg of the genomic DNA and they were reacted at 37°C for 16 hours.

On the other hand, the plasmid pUC19 was completely cleaved with BamHI and ligated thereto the above-obtained genomic DNA fragment with T4DNA ligase to obtain a mixture of various recombinant plasmids.

### Example 4

### To obtain hydantoinase gene derived from Bacillus sp. KNK245

Based on the nucleotide sequences of hydantoinase genes derived form Agrobacterium sp. KNK712 (FERM BP-1900) of Reference Example 1 and Lul220 strain disclosed in JP-A 62-87089, two kinds of primers 1 and 2 (see Table 1) having sequences of each complementary strand of the above nucleotide sequences oriented toward opposite directions from 1155 bp interrupting nucleotides were synthesized. A polymerase chain reaction (PCR) was carried out by using two primers which were synthesized by using genomic DNA derived from Bacillus sp. KNK245 prepared in Example 3 as a template to obtain about 1.2 kbp fragment. The nucleotide sequencing of this fragment was carried out by using DNA sequence kit (manufactured by Applied Biosystems). As a result, it had high complementation to a known hydantoinase gene and it was found that the PCR fragment obtained was a part of the hydantoinase gene. Then, two primers 3 and 4 (Table 1) having oppositely directed sequences of respective complementary strands of this fragment were synthesized.

Further, primer 5 (Table 1) having the sequence of the vector portion in the recombinant DNA of Example 3 was synthesized and, by using the above synthesized primers and the latter primer and using the recombinant DNA of Example 3 as a template, PCR was carried out to obtain two kinds of DNA fragments containing the front half and back half parts of the hydantoinase gene, respectively. These DNA fragments were subjected to nucleotide sequencing. Based on nucleotide sequences expected from the N-terminal amino acid sequence of the hydantoinase protein of Example 2, the translation codon was determined. Together with the results of the above-determined nucleotide sequences, the entire nucleotide sequence of the gene encoding hydantoinase was determined.

Then, based on the nucleotide sequence thus obtained, primer 6 (Table 1) having a sequence about the initiation codon of the hydantoinase gene and NdeI restriction site and primer 7 (Table 1) having a sequence about PstI restriction site in the hydantoinase gene were synthesized and PCR was carried out by using the genomic DNA of Example 3 as a template to obtain a 1.2 kb fragment 1. Then, primer 8 (Table 1) having an oppositely directed sequence at the above PstI restriction site and primer 9 (Table 1) having a sequence corresponding to the downstream of the termination codon and HindIII restriction site were synthesized and PCR was carried out by using the genomic DNA of Example 3 as template to obtain a 0.25 kb fragment 2. Fragment 1 was restricted with NdeI and PstI, fragment 2 was restricted with PstI and HindIII and pUCNT which was an improved vector plasmid of pUC19 described in WO94/03613 was restricted with NdeI and HindIII. The restricted fragments were ligated with T4DNA ligase to obtain plasmid pTH102 containing the hydantoinase gene (see Fig. 2).

**Table 1**

| |
|---|
| Primer 1 |
|            G        A      G T      A               A 5'-AAA( )GGAAC( )AT(T)GT( )ACCGC-3' C      T               T C        C      C |
| Primer 2 |
|       G          G 5'-GCGTC(A)GAACC(A)ACCGCGATCGT-3' T          T C          C |
| Primer 3 |
| 5'-TAACATCCTTTTGCATCAACCACTTC-3' |
| Primer 4 |
| 5'-AAAAAAGGAAGAATCACGTTAAA-3' |
| Primer 5 |
| 5'-GTTTTCCCAGTCACGAC-3' |
| Primer 6 |
| 5'-TACGAGCATATGAAAAAGATCATTAAGAACGG AAC-3' |
| Primer 7 |
| 5'-GTGTGTTTCTGCAGAAATTACCCGTTC-3' |
| Primer 8 |
| 5'-TAATTTCTGCAGAAACACACCATAT-3' |
| Primer 9 |
| 5'-G C T G A A A G C T T C G A A A T A A G C C A T T T T C G A G C A G-3' |
| Primer 10 |
| 5'-TAATTTCTGCAGAAACACACCACATGGCCGT CG-3' |
| Primer 11 |
| 5'-C G A G C A A G C T T C T A C T A A A T G G T T A A T T T C T C A T-3' |
| Primer 12 |
| 5'-GGAAGCTTTCATTAAATGGTTAATTTCTCATC TTCTT-3' |
| Primer 13 |
| 5' -TACGAGGGATCCTATAAATTCCAACAAGTGC TATA-3' |

### Example 5

### Preparation of recombinant DNA for expression of Bacillus sp. KNK245 hydantoinase gene

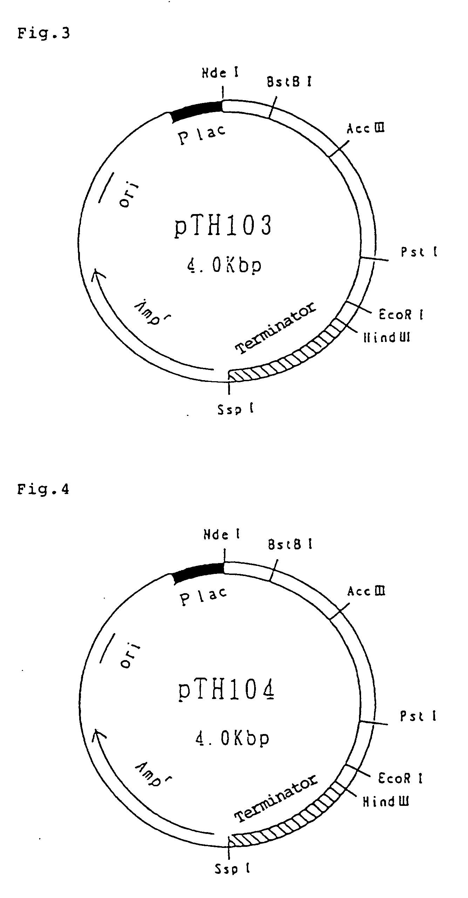

According to the same manner for obtaining fragment 2 in Example 4, PCR was carried out to obtain a 0.25 kb fragment 3 except that, instead of the primer having PstI restriction site used for the production of fragment 2, synthesized primer 10 (Table 1) wherein one nucleotide at NdeI restriction site near PstI restriction site was substituted to block cleavage with NdeI was used and instead of the primer having the sequence about the termination codon, synthesized primer 11 (Table 1) wherein the number of nucleotides at the downstream of the termination codon were further reduced was used. Then, according to the same manner for obtaining pTH1O2 in Example 4, the plasmid pTH1O3 was obtained from the fragments 1 and 3 and pUCNT (see Fig. 3).

Further, according to the same manner for obtaining pTH103, pTH104 was obtained except that, instead of the primer 11 having the downstream sequence about the termination codon, primer 12 (Table 1) was used.

Escherichia coli HB101 transformed with pTH104 was designated as Escherichia coli HB101 pTH104. This strain has been deposited at National Institute of Bioscience and Human-Technology as of November 2, 1994 under the accession number of FERM BP-4864.

### Reference Example 2

### To obtain plasmid containing gene for hydantoinase derived from Pseudomonas sp. KNK003A (FERM BP-3181)

According to the same manner as described in Reference Example 1, it was found that hydantoinase gene was located on the plasmid pPHD301 described in WO92/10579.

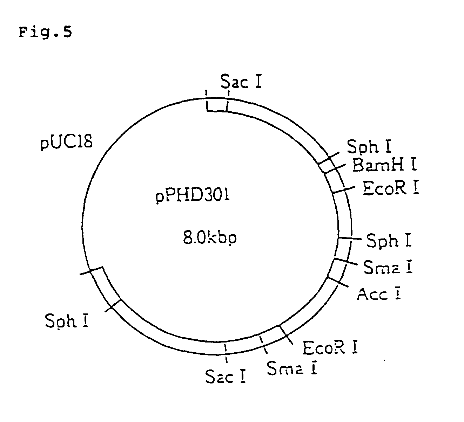

Escherichia coli HB101 was transformed by pPHD301 to obtain Escherichia coli HB101pPHD301 (FERM BP-4866). The restriction enzyme map of pPHD301 is shown in Fig. 5.

The nucleotide sequencing of the hydantoinase gene was carried out according to the same manner as described in Reference Example 1. The results are shown in SEQ No. 2.

### Example 6

### Conversion of 5-(p-hydroxyphenyl)hydantoin into D-N-carbamoyl-(p-hydroxyphenyl)glycine by transformant obtained

The transformant obtained in Example 5, Escherichia coli HB101pTH104 was inoculated into 50 ml of 2YT broth (16 g/liter of polypeptone, 10 g/liter of yeast extract, 5 g/liter of sodium chloride; pH 7.0) containing 100 µg/ml of ampicillin and 400 ppm manganese chloride tetrahydrate and cultivated at 37°C for 24 hours. The microbial cells were collected from 50 ml of this culture broth and suspended in 0.05 M carbonate buffer (pH 8.7) to make up the volume to 50 ml. Triton X-100 was added so that the final concentration was 0.05%. To this was added 1.5 g of 5-(p-hydroxyphenyl)hydantoin and the mixture was allowed to react with stirring at 40°C for 3 hours in the stream of nitrogen with adjusting the pH at 8.7 by 6N sodium hydroxide. After completion of the reaction, the reaction mixture was centrifuged and the supernatant was subjected to a colorimetric determination of D-N-carbamoyl-(p-hydroxyphenyl)glycine therein by color development with 10% solution of p-diemthylaminobenzaldehyde in conc. hydrochloric acid. As a result, D-N-carbamoyl-(p-hydroxyphenyl)glycine was produced in the conversion yield of 97.5%.

The reaction mixture was adjusted to pH 5 with hydrochloric acid. After centrifugation, the supernatant was concentrated, adjusted to pH 2 with conc. hydrochloric acid and stored at 4°C. The precipitated crystals were filtered off and recrystallized from water-ethanol to obtain 970 mg of D-N-carbamoyl-(p-hydroxyphenyl)glycine, m.p. 176-177°C, [α]_{D}²⁰ = -177.0° (c=0.5, 5% ethanol).

### Example 7

### Preparation of immobilized hydantoinase

Microbial cells were collected from 1 liter of a culture broth of Escherichia coli HB101pTH104 obtained according to the same manner as described in Reference Example 2, suspended in aqueous 1 mM manganese sulfate to make up the volume to 60 ml, adjusted to pH 8.5 and disrupted by ultrasonication. To the enzyme solution thus obtained was added 20 g of an anion exchange resin, Duolite A-568 equilibrated to pH 8.5 and the mixture was stirred at 15°C for 20 hours to adsorb the enzyme. To this mixture was added glutaraldehyde so that the final concentration thereof was 0.1% and the mixture was stirred for 1 hour to proceed crosslinking reaction. Then, the resin was collected and washed to obtain 20 g of an immobilized hydantoinase.

### Example 8

### Conversion of 5-(p-hydroxyphenyl)hydantoin into D-carbamoyl-(p-hydroxyphenyl)glycine by immobilized enzyme

Five grams of the immobilized hydantoinase obtained in Example 9 was added to 100 ml of aqueous 1 mM manganese sulfate at 40°C which was bubbled with nitrogen. To this was added 3 g of 5-(p-hydroxyphenyl)hydantoin and the reaction was carried out with stirring at 40°C for 3 hours with bubbling nitrogen. After completion of the reaction, the reaction mixture was allowed to stand and sucked off to collect the reaction mixture. According to the same manner as described in Example 6, the carbamoyl amino acid product was purified to obtain 2.2 g of D-N-carbamoyl-(p-hydroxyphenyl)glycine.

### Example 9

### Expression of Bacillus sp. KNK245 hydantoinase gene in Bacillus subtilis

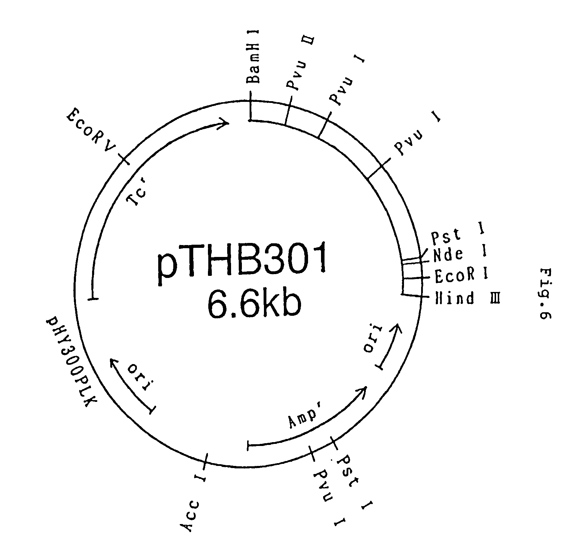

According to the same manner as described in Example 4, PCR was carried out by using the genomic DNA of Bacillus sp. KNK245 as a template and using primers 12 and 13 (Table 1) to obtain a 1.7 kb fragment. This was cleaved with BamHI and HindIII and ligated to the plasmid pHY300PLK (Takara Shuzo) which had been cleaved in a similar manner to obtain the plasmid pTHB301. Its restriction enzyme map is shown in Fig. 6.

This plasmid pTHB301 was introduced into Bacillus subtilis ISW1214 (available from Takara Shuzo), Bacillus subtilis YS-11 (IAM12019) or Bacillus subtilis 3115 (IAM12020) by electroporation (using Shimadzu Corporation GTE-10 Model, 10 KV/cm, 2 ms) and cultivated in the culture medium as shown in Example 3 to which 0.02 g/liter of manganese chloride tetrahydrate was added at 37°C for 20 hours. A cell-free extraction was prepared by collecting the microbial cells and disrupting them with ultrasonication. As a result, each hydantoinase activity was about 2.2. times higher than that obtained by cultivation of Bacillus sp. KNK245 under the same conditions.

### Example 10

### Expression of Bacillus sp. KNK245 hydantoinase gene in thermophile

Bacillus stearothermophilus IFO12550 was transformed with the plasmid pTHB301 prepared in Example 9 by protoplast method according to J. Bacteriol., 149, 824 (1982) and cultivated according to the same manner as described in Example 11 to prepare a cell-free extract. The hydantoinase activity was about 3.6 times higher than that of Bacillus sp. KNK245 prepared under the same conditions.

As described hereinabove, according to the present invention, a microorganism having very high hydantoinase activity can be created and, by using this as a source of enzyme, D-N-carbamoyl-α-amino acids can be produced, efficiently.

### Deposit of Microorganisms

Agrobacterium sp. KNK712 has been deposited as of May 31, 1988 under the accession number of FERM BP-1900; Pseudomonas sp. KNK003A has been deposited as of December 1, 1990 under the accession number of FERM BP-3181; Bacillus sp. KNK245 has been deposited as of November 2, 1994 under the accession number of FERM BP-4863; and Escherichia coli HB101 pAH1043 has been deposited as of November 2, 1994 under the accession number of FERM BP-4866 at the following Depsoitary Authority under Budapest Treaty, respectively.
Name: National Institute of Bioscience and Human-Technology (formerly Fermentation Research Institute), Agency of Industrial Science & Technology, Ministry of International Trade & Industry
Address: 1-3, Higashi 1 chome Tsukuba-shi Ibaraki-ken 305, Japan

### Sequence Listing (Substituted)

SEQ ID NO: 1
   SEQUENCE LENGTH: 2105
   SEQUENCE TYPE: nucleic acid
   SEQUENCE: 
10 20 30 40 50 60
GTCGACTTCAACTCGTTCCGCCTCGAACGAGAGCGCTCTGCACAGAATCGTCTTCGCTAG

70 80 90 100 110 120
GCCGCCGTTCCGCTTGACAGCTTCAAAACACCATGCAAACTTGTATAAAGAATTACATTC

130 140 150 160 170 180
CATATACGGAACAAACTCTTCAATCCTGAGAGCGACATCATGGACATCATTATCAAAAAC
MetAspIleIleIleLysAsn

190 200 210 220 230 240
GGAACCATCGTGACCGCGGATGGCATTTCTCGCGCCGATCTCGGGATCAAGGATGGCAAG
GlyThrIleValThrAlaAspGlyIleSerArgAlaAspLeuGlyIleLysAspGlyLys
 
250 260 270 280 290 300
ATCACCCAGATCGGCGGCGCTCGGCCCAGCGGAGCGGACGATCGACGCGGCCGGCCGC
IleThrGlnIleGlyGlyAlaLeuGlyProAlaGluArgThrIleAspAlaAlaGlyArg

310 320 330 340 350 360
TACGTCTTTCCGGGCGGCATAGACGTTCACACGCATGTCGAAACGGTCAGCTTCAACACG
TyrValPheProGlyGlyIleAspValHisThrHisValGluThrValSerPheAsnThr

370 380 390 400 410 420
CAGTCGGCCGACACGTTCGCAACAGCGACGGTCGCGGCCGCCTGTGGCGGAACGACAACC
GlnSerAlaAspThrPheAlaThrAlaThrValAlaAlaAlaCysGlyGlyThrThrThr

430 440 450 460 470 480
ATCGTCGATTTCTGTCAGCAGGATCGCGGCCACAGCCTGGCGGAACCCGTCCCCAAGTGG
IleValAspPheCysGlnGlnAspArgGlyHisSerLeuAlaGluProValProLysTrp

490 500 510 520 530 540
GACGGTATGGCCGGCGGCAAGTCGGCGATCGATTACGGCTACCACATCATCGTGCTCGAC
AspGlyMetAlaGlyGlyLysSerAlaIleAspTyrGlyTyrHisIleIleValLeuAsp

550 560 570 580 590 600
CCGACCGACAGCGTGATTGAGGAGCTGGAGGTGCTTCCCGATCTTGGCATTACCTCCTTC
ProThrAspSerValIleGluGluLeuGluValLeuProAspLeuGlyIleThrSerPhe
 
610 620 630 640 650 660
AAGGTCTTCATGGCCTATCGCGGCATGAACATGATCGACGACCTGACGCTGCTGAAGACG
LysValPheMetAlaTyrArgGlyMetAsnMetIleAspAspValThrLeuLeuLysThr

670 680 690 700 710 720
CTCGACAAGGCGGTCAAGACCGGATCGCTCGTCATGGTGCACGCGGAAAACGGCGACGCC
LeuAspLysAlaValLysThrGlySerLeuValMetValHisAlaGluAsnGlyAspAla

730 740 750 760 770 780
GCCGACTATCTGCGCGACAAGTTCGTGGCCGAGGGCAAAACCGCGCCGATCTACCACGCG
AlaAspTyrLeuArgAspLysPheValAlaGluGlyLysThrAlaProIleTyrHisAla

790 800 810 820 830 840
CTCAGCCGCCCGCCCCGGGTCGAAGCCGAGGCAACCGCGCGGGCCCTCGCCCTGGCCGAA
LeuSerArgProProArgValGluAlaGluAlaThrAlaArgAlaLeuAlaLeuAlaGlu

850 860 870 880 890 900
ATCGTCAACGCCCCGATCTACATAGTCCATGTGACCTGCGAGGAGTCCCTTGAGGAGGTG
IleValAsnAlaProIleTyrIleValHisValThrCysGluGluSerLeuGluGluVal

910 920 930 940 950 960
ATGCGCGCAAAATCGCGAGGCGTCCGCGCTCTGGCGGAAACCTGCACGCATTACCTTTAC
MetArgAlaLysSerArgGlyValArgAlaLeuAlaGluThrCysThrHisTyrLeuTyr
 
970 980 990 1000 1010 1020
CTCACCAAGGAAGACCTGGAGCGGCCGGATTTCGAAGGTGCGAAATACGTTTTCACACCG
LeuThrLysGluAspLeuGluArgProAspPheGluGlyAlaLysTyrValPheThrPro

1030 1040 1050 1060 1070 1080
CCGGCCCGCGCGAAGAAAGACCATGACGTTCTCTGGAACGCACTCAGAAACGGTGTGTTC
ProAlaArgAlaLysLysAspHisAspValLeuTrpAsnAlaLeuArgAsnGlyValPhe

1090 1100 1110 1120 1130 1140
GAAACGGTTTCCTCCGACCATTGCTCCTGGCTCTTCAAGGGGCACAAGGACCGGGGCCGG
GluThrValSerSerAspHisCysSerTrpLeuPheLysGlyHisLysAspArgGlyArg

1150 1160 1170 1180 1190 1200
AACGACTTTCGCGCCATCCCGAACGGCGCGCCGGGCGTCGAGGAACGGTTGATGATGGTC
AsnAspPheArgAlaIleProAsnGlyAlaProGlyValGluGluArgLeuMetMetVal

1210 1220 1230 1240 1250 1260
TATCAGGGCGTCAACGAAGGCCGGATTTCCCTTACCCAGTTCGTGGAACTGGTCGCCACG
TyrGlnGlyValAsnGluGlyArgIleSerLeuThrGlnPheValGluLeuValAlaThr

1270 1280 1290 1300 1310 1320
CGCCCGGCCAAGGTCTTCGGAATGTTTCCGCAAAAGGGGACGATCGCGGTCGGTTCGGAC
ArgProAlaLysValPheGlyMetPheProGlnLysGlyThrIleAlaValGlySerAsp
 
1330 1340 1350 1360 1370 1380
GCCGACATCGTCCTTTGGGACCCCGAGGCCGAAATGGTGATCGAACAGACCGCCATGCAC
AlaAspIleValLeuTrpAspProGluAlaGluMetValIleGluGlnThrAlaMetHis

1390 1400 1410 1420 1430 1440
AACGCCATGGATTACTCCTCCTACGAGGGACACAAGGTCAAGGGCGTGCCGAAGACGGTG
AsnAlaMetAspTyrSerSerTyrGluGlyHisLysValLysGlyValProLysThrVal

1450 1460 1470 1480 1490 1500
CTCCTGCGTGGCAAGGTTATCGTCGACGAAGGTTCCTATGTCGGCGAACCGACGGACGGG
LeuLeuArgGlyLysValIleValAspGluGlySerTyrValGlyGluProThrAspGly

1510 1520 1530 1540 1550 1560
AAATTCCTGAAACGTCGCAAATACAAGCAGTAAACCGGCATTCTACGAGAACGATGATGA
LysPheLeuLysArgArgLysTyrLysGln***

1570 1580 1590 1600 1610 1620
CAATACACACTTCGACAAGTGTCGTCGCCTGACGGCGACAGCGTTGCAAGCGGCTTCG

1630 1640 1650 1660 1670 1680
ACAGGCGTTACGGCGCACCGACATTCAATCAGATACCATCGGGCGAACGGCTGGTGTCGG

1690 1700 1710 1720 1730 1740
ACTGCATCGCGCTGATGCTGAAGCACCGTTCCGTCAGACGCTACACGGGGGAACCTCTCC
 
1750 1760 1770 1780 1790 1800
CCCCAGGCACGCTGGAAATGCTGATGGCGGCGGGTCAGTCGGCTGCGACATCCTCCAACA

1810 1820 1830 1840 1850 1860
TGCAGACCGTGTCGGTCATCGCGGTGACGGACCCCGAGAAAAAGACCCGTCTCGCGAAGA

1870 1880 1890 1900 1910 1920
CATGCGCCGGGCAGGACTTCATCGCCAACGCTCCTGTGCTCCTGTGTTTCGTGACGGATC

1930 1940 1950 1960 1970 1980
TTGCGCGCCCCGCCAGGGTCGCCTCCACGATTGGCGCCGATCTTTTCGCGTTGCCGATGA

1990 2000 2010 2020 2030 2040
TCGACACCTTTCTGGCGTCCATCAGCGATTGTTCCATCTTCGCGCAAAACCTTGTTCTCG

2050 2060 2070 2080 2090 2100
CCGCGGAATCGCTCGGCATGGGCACCTGCTATGTCGGAGCCTGCGCAACCGGCCTGATC

CCGGG
SEQ ID NO: 2
   SEQUENCE LENGTH: 1569
   SEQUENCE TYPE: nucleic acid
   SEQUENCE:

10 20 30 40 50 60

TCGGAACCTGAAAACGAAATATCCCGCCGGACGGTGGAAGGTGAAAGGAGGAGTCTCCA

Met

70 80 90 100 110 120

TGACAACAGTTATCAAGGGTGGAACGATCGTCGCCGCCGATCGCAGCTATGAAGCCGATA

ThrThrValIleLysGlyGlyThrIleValAlaAlaAspArgSerTyrGluAlaAspIle

130 140 150 160 170 180

TCCTGATCGAAGGCGAAAAGATCGCCCAGATCGGCAGGGATCTGCAGGGCGACAAGATTG

LeuIleGluGlyGluLysIleAlaGlnIleGlyArgAspLeuGlnGlyAspLysIleVal

190 200 210 220 230 240

TAGACGCCGAGGGTGCCTATATCATTCCCGGCGGCATCGATCCTCACACGCATCTTGAAA

AspAlaGluGlyAlaTyrIleIleProGlyGlyIleAspProHisThrHisLeuGluMet

250 260 270 280 290 300

TGCCCTTTATGGGCACGACCACTGCCGAGACCTGGGAGTCCGGCACTTTCGCGGCTCTCT

ProPheMetGlyThrThrThrAlaGluThrTrpGluSerGlyThrPheAlaAlaLeuSer

310 320 330 340 350 360

CCGGCGGCACCACGATGGTTGTGGATTTCGTCATTCCCGGGCCGGCGGGAATGCTTGCCG

GlyGlyThrThrMetValValAspPheValIleProGlyProAlaGlyMetLeuAlaAla

370 380 390 400 410 420

CCTTCGATCAATGGCAGGAGAGGGCAGCGCGCCAGGCTTCGTCCGACTACTCGCTGCATA

PheAspGlnTrpGlnGluArgAlaAlaArgGlnAlaSerSerAspTyrSerLeuHisMet

430 440 450 460 470 480

TGTGCGTGACCGGCTGGTCAAAGCAGATCTTCGAGGACATGGCGAAGGTGGTCGAGCGCG

CysValThrGlyTrpSerLysGlnIlePheGluAspMetAlaLysValValGluArgGly

490 500 510 520 530 540

GTGTCAACACCTTCAAGCATTTCATGGCCTATAAGGGCGCGCTGATGGTGAACGACGACG

ValAsnThrPheLysHisPheMetAlaTyrLysGlyAlaLeuMetValAsnAspAspGlu

550 560 570 580 590 600

AGATGTTCGCCTCCTTCCAGCGCTGCGCCGCCCTGGGCGCCCTCCCGCTCGTGCATGCGG

MetPheAlaSerPheGlnArgCysAlaAlaLeuGlyAlaLeuProLeuValHisAlaGlu

610 620 630 640 650 660

AAAACGGCGACATCGTCGCCTCGCTGCAGCAGAAATATATGCAGGAAGGCCTCACGGGTC

AsnGlyAspIleValAlaSerLeuGlnGlnLysTyrMetGlnGluGlyLeuThrGlyPro

670 680 690 700 710 720

CGGAGGCTCATGCCTATTCCCGCCCGCCGGAGGTCGAGGGCGAGGCCACCAACCGCGCCA

GluAlaHisAlaTyrSerArgProProGluValGluGlyGluAlaThrAsnArgAlaIle

730 740 750 760 770 780

TCATGATCGCCGATGCCGCAGGCGTGCCGGTCTATATCGTGCATGTCTCCTGTGAACAGG

MetIleAlaAspAlaAlaGlyValProValTyrIleValHisValSerCysGluGlnAla

790 800 810 820 830 840

CGCACGAAGCGATCCGCCGCGCACGCCAGAAGGGAATGCGCGTCTATGGCGAGCCGCTCA

HisGluAlaIleArgArgAlaArgGlnLysGlyMetArgValTyrGlyGluProLeuIle

850 860 870 880 890 900

TCCAGCACCTGCTGCTTGACGAGTCCGAGTACAAGAGAGCCGACTGGGACGAGGCGGCTC

GlnHisLeuLeuLeuAspGluSerGluTyrLysArgAlaAspTrpAspGluAlaAlaArg

910 920 930 940 950 960

GGCGGGTGATGTCCGCGCCATTCCGCGATAAAAGCCATCAGGACAGCCTCTGGCTGGGC

ArgValMetSerAlaProPheArgAspLysSerHisGlnAspSerLeuTrpAlaGlyLeu

970 980 990 1000 1010 1020

TCGCAGCGGGCTCGCTGCAGGTCGTGGCAACGGACCACTGCGCCTTTACCACTGAACAGA

AlaAlaGlySerLeuGlnValValAlaThrAspHisCysAlaPheThrThrGluGlnLys

1030 1040 1050 1060 1070 1080
AGCGGCTGGGACTCAACGACTTCACGAAAATCCCGAACGGCACCGGGGGTTAGAGGACC
ArgLeuGlyLeuAsnAspPheThrLysIleProAsnGlyThrGlyGlyLeuGluAspArg

1090 1100 1110 1120 1130 1140
GGTTGTCGCTGCTCTGGACCTATGGCGTGAAGACGGGGCGGCTCACGCCTAACGAATTCG
LeuSerLeuLeuTrpThrTyrGlyValLysThrGlyArgLeuThrProAsnGluPheVal

1150 1160 1170 1180 1190 1200
TCGCCGTCACCTCCACCAATATCGCACAAATCCTGAACATCTATCCGCAAAAGGGCGCAA
AlaValThrSerThrAsnIleAlaGlnIleLeuAsnIleTyrProGlnLysGlyAlaIle

1210 1220 1230 1240 1250 1260
TCCTGCCTGGGTCCGATGCCGACCTCGTCGTCTGGGATCCAGCCAAATCGAAGAAGATCA
LeuProGlySerAspAlaAspLeuValValTrpAspProAlaLysSerLysLysIleThr

1270 1280 1290 1300 1310 1320
CTGCTTCCGCTCAGAAATCCATCATCGACTACAATATCTTCGAGGGCTACGAGGTGACCG
AlaSerAlaGlnLysSerIleIleAspTyrAsnIlePheGluGlyTyrGluValThrGly

1330 1340 1350 1360 1370 1380
GCATGCCGCGATACACGCTCTCACGCGGCGAGGTCGTATGGGGCGAGGAAGGCAGCAACG
MetProArgTyrThrLeuSerArgGlyGluValValTrpGlyGluGluGlySerAsnGlu

1390 1400 1410 1420 1430 1440
AACCGCGGCCCGGTCGCGGTAGGTTCGTCGAGCGCCGGCCCTTTGCCGCCGTCAGCCGTG
ProArgProGlyArgGlyArgPheValGluArgArgProPheAlaAlaValSerArgAla

1450 1460 1470 1480 1490 1500
CCCTTTCAACCTGGAAGGAGATCGTCGCTCCGCGTGCGGTGGAGCGCTCGGCAAAGCATA
LeuSerThrTrpLysGluIleValAlaProArgAlaValGluArgSerAlaLysHisMet

1510 1520 1530 1540 1550 1560
TGCCGATCGGCGTTTGAATTTTGGGAGGCAGGCTCGAACTACGGTTTGGCCTGTCTCAGG
ProIleGlyVal***

ACAATCCAT

## Claims

1. A transformant microorganism obtainable by transforming a host microorganism with a recombinant DNA of a DNA fragment containing a gene encoding an enzyme which converts 5-substituted hydantoins into corresponding D-N-carbamoyl-α-amino acids by asymmetric cleavage hydrolysis (hereinafter referred to as hydantoinase) derived from Bacillus sp. KNK245 (FERM BP-4863) and a vector DNA.

2. A transformant microorganism according to claim 1, wherein the host microorganism is selected from a microorganism belonging to the genus Escherichia, Pseudomonas, Flavobacterium, Bacillus, Serratia, Agrobacterium, Corynebacterium or Brevibacterium.

3. A transformant microorganism according to claim 2, wherein the transformant microorganism is Escherichia coli HB101 pTH102, Escherichia coli HB101 pTH103, Escherichia coli HB101 pTH104 (FERM BP-4864).

4. A process for producing an enzyme which converts 5-substituted hydantoins into corresponding D-N-carbamoyl-α-amino acids by asymmetric cleavage hydrolysis thereof which comprises using the transformant microorganism according to any one of claims 1 to 3.

5. A process for producing D-N-carbamoyl-α-amino acids which comprises using the enzyme obtained by a process according to claim 4.

6. A process according to claim 5, wherein the 5-substituted hydantoin is a compound represented by the general formula (1) : 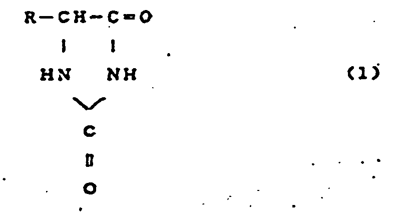 wherein R is phenyl, phenyl substituted with hydroxy, alkyl, substituted alkyl, aralkyl or thienyl.

7. A recombinant plasmid obtained by recombination of a DNA fragment encoding hydantoinase derived from Bacillus sp. KNK245 (FERM BP-4863) and plasmid pUCNT having the restriction enzyme map of any of Figs. 2 to 4.

8. A gene for a protein having an enzymatic activity for conversion of 5-substituted hydantoins into-corresponding D-N-carbamoyl-α-amino acids by asymmetric cleavage hydrolysis thereof, wherein the protein is derivable from Bacillus sp KNK245 (FERM BP-4863).

9. An enzyme protein obtainable by the method of claim 4.

## Patentansprüche

1. Transformierter Mikroorganismus, erhältlich durch Transformieren eines Wirtsmikroorganismus mit einer rekombinanten DNA eines DNA-Fragments, enthaltend ein Gen, das ein Enzym codiert, das 5-substituierte Hydantoine durch asymmetrische hydrolytische Spaltung in die korrespondierenden D-N-Carbamoyl-α-aminosäuren umwandelt (nachfolgend als "Hydantoinase" bezeichnet), abgeleitet aus Bacillus sp. KNK245 (FERM BP-4863), und eine Vektor-DNA.

2. Transformierter Mikroorganismus gemäss Anspruch 1, worin der Wirtsmikroorganismus ausgewählt ist aus einem Mikroorganismus des Genus Escherichia, Pseudomonas, Flavobacterium, Bacillus, Serratia, Agrobacterium, Corynebacterium oder Brevibacterium.

3. Transformierter Mikroorganismus gemäss Anspruch 2, worin der transformierte Mikroorganismus Escherichia coli HB101 pTH102, Escherichia coli HB101 pTH103 oder Escherichia coli HB101 pTH104 (FERM BP-4864) ist.

4. Verfahren zur Herstellung eines Enzyms, das 5-substituierte Hydantoine durch asymmetrische hydrolytische Spaltung derselben in die korrespondierenden D-N-Carbamoyl-α-aminosäuren umwandelt, umfassend die Verwendung des transformierten Mikroorganismus gemäss einem der Ansprüche 1 bis 3.

5. Verfahren zur Herstellung von D-N-Carbamoyl-α-aminosäuren, umfassend die Verwendung des Enzyms, erhalten durch ein Verfahren gemäss Anspruch 4.

6. Verfahren gemäss Anspruch 5, worin das 5-substituierte Hydantoin eine Verbindung, dargestellt durch die allgemeine Formel (1), ist: 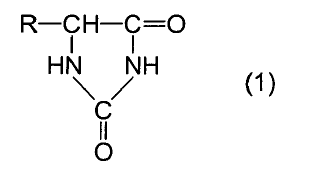 worin R Phenyl, Hydroxy-substituiertes Phenyl, Alkyl, substituiertes Alkyl, Aralkyl oder Thienyl ist.

7. Rekombinantes Plasmid, erhalten durch Rekombination eines DNA-Fragments, codierend Hydantoinase, abgeleitet von Bacillus sp. KNK245 (FERM BP-4863) und Plasmid pUCNT, mit der Restriktionsenzymkarte einer der Figuren 2 bis 4.

8. Gen für ein Protein mit der enzymatischen Aktivität zur Umwandlung von 5-substituierten Hydantoinen durch asymmetrische hydrolytische Spaltung derselben in korrespondierende D-N-Carbamoyl-α-aminosäuren, wobei das Protein aus Bacillus sp. KNK245 (FERM BP-4863) erhältlich ist.

9. Enzymprotein erhältlich durch das Verfahren gemäß Anspruch 4.

## Revendications

1. Microorganisme transformant susceptible d'être obtenu par transformation d'un microorganisme hôte avec un ADN recombinant d'un fragment d'ADN contenant un gène codant une enzyme qui convertit des hydantoïnes substituées en position 5 en acides D-N-carbamoyl-α-aminés correspondants par hydrolyse à clivage asymétrique (à laquelle on se réfère ci-dessous par hydantoïnase), dérivée de Bacillus sp. KNK245 (FERM BP-4863) et un vecteur d'ADN.

2. Microorganisme transformant selon la revendication 1, le microorganisme hôte étant sélectionné parmi des microorganismes appartenant aux genres Escherichia, Pseudomonas, Flavobacterium, Bacillus, Serratia, Agrobacterium, Corynebacterium ou Brevibacterium.

3. Microorganisme transformant selon la revendication 2, lequel microorganisme transformant est Escherichia coli HB101 pTH102, Escherichia coli HB101 pTH103, Escherichia coli HB101 pTH104 (FERM BP-4864).

4. Procédé pour produire une enzyme qui convertit des hydantoïnes substituées en position 5 en acides D-N-carbamoyl-α-aminés correspondants par hydrolyse à clivage asymétrique de celles-ci, qui comprend l'utilisation du microorganisme transformant selon l'une quelconque des revendications 1 à 3.

5. Procédé pour produire des acides D-N-carbamoyl-α-aminés, qui comprend l'utilisation de l'enzyme obtenue par un procédé selon la revendication 4.

6. Procédé selon la revendication 5, dans lequel l'hydantoïne substituée en position 5 est un composé représenté par la formule générale (1) : 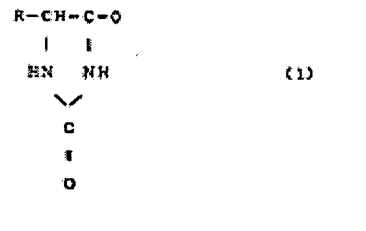 dans laquelle R représente un groupe phényle, phényle substitué par un groupe hydroxy, alkyle, alkyle substitué, aralkyle ou thiényle.

7. Plasmide recombinant obtenu par recombinaison d'un fragment d'ADN codant une hydantoïnase dérivée de Bacillus sp. KNK245 (FERM BP-4863) et du plasmide pUCNT ayant la carte de restriction enzymatique de l'une quelconque des figures 2 à 4.

8. Gène pour une protéine ayant une activité enzymatique pour la conversion d'hydantoïnes substituées en position 5 en acides D-N-carbamoyl-α-aminés correspondants par hydrolyse à clivage asymétrique de celles-ci, la protéine étant dérivable de Bacillus sp. KNK245 (FERM BP-4863).

9. Protéine enzyme susceptible d'être obtenue par le procédé de la revendication 4.
